# EUROPÄISCHE PATENTSCHRIFT

(11) **EP 2 162 421 B1**
(45) Veröffentlichungstag und Bekanntmachung des Hinweises auf die Patenterteilung: **26.11.2014**
(21) Anmeldenummer: 08750177.1
(22) Anmeldetag: 08.05.2008
(51) Int. Cl.: C07C 67/03, C07C 69/54

(54) **VERFAHREN ZUR HERSTELLUNG VON BUTANDIOLDIMETHACRYLATEN**
METHOD FOR PRODUCING BUTANEDIOL DIMETHACRYLATES
PROCÉDÉ DE PRODUCTION DE DIMÉTHACRYLATES DE BUTANEDIOL

(30) Priorität: 05.07.2007 DE 102007031474
(43) Veröffentlichungstag der Anmeldung: 17.03.2010
(73) Patentinhaber: Evonik Röhm GmbH, 64293 Darmstadt (DE)
(72) Erfinder: KNEBEL, Joachim, 64665 Alsbach-Hähnlein (DE); SCHÜTZ, Thorben, 64342 Seeheim-Jugenheim (DE); TRAUTHWEIN, Harald, 68642 Bürstadt (DE); KEHR, Thomas, 64367 Muehltal (DE); LAUSTER, Günter, 67547 Worms (DE); PROTZMANN, Guido, 64625 Bensheim (DE); KÖLBL, Gerhard, 64579 Gernsheim (DE); WESTHÄUSER, Günter, 67583 (DE)
(86) Internationale Anmeldenummer: PCT/EP2008/055666
(87) Internationale Veröffentlichungsnummer: WO 2009/003743

(56) Entgegenhaltungen:
- EP-A- 0 534 666
- US-A- 4 672 105

## Beschreibung

Die vorliegende Erfindung betrifft Verfahren zur Herstellung von Butandioldimethacrylaten.

Butandioldimethacrylate werden vielfach als Comonomere eingesetzt. Dementsprechend sind vielfältige Methoden bekannt, um diese Verbindungen zu erhalten. Zur Verbesserung der Ausbeute und der Selektivität der Reaktion können unterschiedliche Katalysatoren eingesetzt werden.

Beispielsweise beschreibt die Druckschrift DE 28 05 702 die Herstellung von Estern ungesättigter Carbonsäuren. Zur Katalyse der beschriebenen Reaktionen können insbesondere Verbindungen eingesetzt werden, die Zirkonium und/oder Calcium enthalten. Zu den besonders geeigneten Katalysatoren gehört insbesondere Zirkoniumacetylacetonat. Die Herstellung von 1,3-Butandioldimethacrylat wird explizit beschrieben. Die Umsetzungen führen zu hohen Ausbeuten von ca. 97%, bezogen auf den eingesetzten Alkohol. Nachteilig ist jedoch, dass der Katalysator relativ teuer ist und nur sehr schwer aus der Reaktionsmischung abgetrennt werden kann.

Ein Verfahren zur Abtrennung dieses Katalysators wird zwar in DE 199 40 622 dargelegt, jedoch ist das Verfahren in der Durchführung relativ teuer.

Des Weiteren können Säuren oder Basen eingesetzt werden, um die Umesterung zu katalysieren. Derartige Reaktionen werden beispielsweise in CN 1355161, DE 34 23 443 oder EP-A-0 534 666 dargelegt. Bei Einsatz dieser Katalysatoren muss allerdings mit Nebenreaktionen gerechnet werden, wie beispielsweise der Michael-Addition, die sowohl die Reinheit des gewünschten Dimethacrylats als auch die Ausbeute schmälert.

In Anbetracht des Standes der Technik war es nun Aufgabe der vorliegenden Erfindung, ein Verfahren zur Herstellung von Butandioldimethacrylaten zur Verfügung zu stellen, bei dem das Produkt sehr kostengünstig erhalten werden kann. Darüber hinaus sollte das erhaltene Butandioldimethacrylat nur sehr geringe Mengen an Nebenprodukten und Katalysatorresten enthalten.

Eine weitere Aufgabe der Erfindung bestand darin, ein Verfahren zu schaffen, bei dem Butandioldimethacrylat sehr selektiv erhalten werden kann.

Darüber hinaus war es Aufgabe der vorliegenden Erfindung, Verfahren zur Herstellung von Butandioldimethacrylaten zur Verfügung zu stellen, die einfach und kostengünstig durchgeführt werden können. Hierbei sollte das Produkt möglichst in hohen Ausbeuten und, insgesamt gesehen, unter geringem Energieverbrauch erhalten werden.

Gelöst werden diese sowie weitere nicht explizit genannte Aufgaben, die jedoch aus den hierin einleitend diskutierten Zusammenhängen ohne Weiteres ableitbar oder erschließbar sind, durch Verfahren mit allen Merkmalen des Patentanspruchs 1. Zweckmäßige Abwandlungen der erfindungsgemäßen Verfahren werden in den auf Anspruch 1 rückbezogenen abhängigen Ansprüchen unter Schutz gestellt.

Gegenstand der vorliegenden Erfindung ist dementsprechend ein Verfahren zur Herstellung von Butandioldimethacrylaten, umfassend die Umesterung von Butandiol mit einem Ester der Methacrylsäure in Gegenwart von Katalysatoren, wobei als Katalysator eine Kombination eingesetzt wird, die mindestens eine Lithiumverbindung und mindestens eine Calciumverbindung umfasst, mindestens eine der Verbindungen des Lithiums und/oder des Calciums ein Oxid, ein Hydroxid, ein Alkoxid mit 1 bis 4 Kohlenstoffatomen oder ein Carboxylat mit 1 bis 4 Kohlenstoffatomen ist und zumindest ein Teil der Umsetzung in Gegenwart einer wirksamen Menge an Wasser erfolgt, wobei das Wasser der Reaktionsmischung himzu gegeben wird.

Hierdurch gelingt es auf nicht vorhersehbare Weise ein Verfahren zur Herstellung von Butandioldimethacrylaten zur Verfügung zu stellen, bei dem das Produkt sehr kostengünstig erhalten wird. Überraschend enthält das erhaltene Produkt nur sehr geringe Mengen an Nebenprodukten und Katalysatorresten.

Des Weiteren ermöglicht das erfindungsgemäße Verfahren eine besonders selektive Herstellung von Butandioldimethacrylaten.

Weiterhin kann das erfindungsgemäße Verfahren einfach und kostengünstig durchgeführt werden, wobei das Produkt in hohen Ausbeuten und, insgesamt gesehen, unter geringem Energieverbrauch erhalten werden kann.

Erfindungsgemäß wird mindestens ein Butandioldimethacrylat hergestellt. Erfindungsgemäß kann 1,3-Butandioldimethacrylat (2-Methylpropensäure-1,3-butandiyldiester), 1,4-Butandioldimethacrylat (2-Methylpropensäure-1,4-butandiyldiester) oder eine Mischung umfassend 1,3-Butandioldimethacrylat und 1,4-Butandioldimethacrylat hergestellt werden. Beide Verbindungen sind seit langem bekannt , wobei 1,3-Butandioldimethacrylat die CAS-Nummer 1189-08-8 und 1,4-Butandioldimethacrylat die CAS-Nummer 2082-81-7 besitzt.

Zur Herstellung von Butandioldimethacrylat wird erfindungsgemäß 1,3-Butandiol und/oder 1,4-Butandiol eingesetzt. Die Verbindungen sind kommerziell zum Beispiel von BASF AG oder Celanese AG erhältlich. Die CAS-Nummer von 1,3-Butandiol ist 107-88-0, die von 1,4-Butandiol 110-63-4.

Gemäß der vorliegenden Erfindung wird Butandiol mit einem Ester der Methacrylsäure umgesetzt. Besonders geeignete Methacrylate werden insbesondere von Alkoholen mit 1 bis 4 Kohlenstoffatomen gebildet. Zu diesen gehören insbesondere Methanol, Ethanol, n-Propanol, iso-Propanol, n-Butanol und tert.-Butanol. Besonders bevorzugt wird insbesondere Ethylmethacrylat oder Methylmethacrylat eingesetzt, wobei Methylmethacrylat ganz besonders bevorzugt ist.

Das Gewichtsverhältnis von Butandiol zum Ester der Methacrylsäure liegt vorzugsweise im Bereich von 1:2 bis 1:20, besonders bevorzugt 1:3 bis 1:10 und ganz besonders bevorzugt im Bereich von 1:4 bis 1:8.

Erfindungsgemäß wird zur Katalyse der vorliegenden Umesterung eine Kombination eingesetzt, die mindestens eine Lithiumverbindung und mindestens eine Calciumverbindung umfasst, wobei mindestens eine der Verbindungen des Lithiums und/oder des Calciums ein Oxid, ein Hydroxid, ein Alkoxid mit 1 bis 4 Kohlenstoffatomen oder ein Carboxylat mit 1 bis 4 Kohlenstoffatomen ist. Vorzugsweise umfasst der Katalysator mindestens eine Lithiumverbindung ausgewählt aus der Gruppe aus Lithiumoxid (Li₂O), Lithiumhydroxid (LiOH), Lithiumalkoholat mit 1 bis 4 Kohlenstoffatomen, wie Lithiummethanolat (Li(CH₃O)), Lithiumethanolat (Li(CH₃CH₂O)) und/oder Lithiumcarboxylat mit 1 bis 4 Kohlenstoffatomen, wie beispielsweise Lithiumacetat, und mindestens eine Calciumverbindung ausgewählt aus der Gruppe aus Calciumoxid (CaO), Calciumhydroxid (Ca(OH)₂), Calciumalkoholat mit 1 bis 4 Kohlenstoffatomen, wie Calciummethanolat (Ca(CH₃O)₂), Calciumethanolat (Ca(CH₃CH₂O)₂) und/oder Calciumcarboxylat mit 1 bis 4 Kohlenstoffatomen, wie beispielsweise Calciumacetat.

Die Verbindungen des Lithiums und/oder des Calciums können vorzugsweise basischer Natur sein, d.h. beim Auflösen in Wasser erfolgt eine Erhöhung des pH-Wertes.

Zweckmäßig kann der Katalysator als Lithiumverbindung zum Beispiel Lithiumhydroxid (LiOH), Lithiumoxid (Li₂O), Lithiummethanolat (Ca(CH₃O)₂) und/oder Lithiumethanolat (Li(CH₃CH₂O)) enthalten.

Von besonderem Interesse sind des Weiteren Katalysatoren, die als Calciumverbindung Calciumoxid (CaO), Calciumhydroxid (Ca(OH)₂), Calciummethanolat (Ca(CH₃O)₂) und/oder Calciumethanolat (Ca(CH₃CH₂O)₂) umfassen.

Vorzugsweise kann als Katalysator eine Mischung eingesetzt werden, die Lithiumhydroxid und Calciumoxid oder Lithiumhydroxid und Calciumhydroxid umfasst.

Das Verhältnis des Gewichts der Lithiumverbindung zum Gewicht der Calciumverbindung kann je nach Reaktionsbedingungen in einem weiten Bereich liegen. Zweckmäßig kann dieses Verhältnis beispielsweise im Bereich von 20:1 bis 1:20, besonders bevorzugt im Bereich von 1:1 bis 1:10 liegen.

Die Menge an eingesetztem Katalysator kann in einem weiten Bereich liegen. Von besonderem Interesse sind jedoch Verfahren, bei denen der Anteil an Katalysator, bezogen auf das Gewicht des eingesetzten Butandiols, im Bereich von 0,05 bis 8 Gew.-%, vorzugsweise im Bereich von 0,01 bis 5 Gew.-% und besonders bevorzugt im Bereich von 0,1 bis 1 Gew.-% liegt.

Erfindungsgemäß erfolgt die Umsetzung in Gegenwart einer wirksamen Menge an Wasser. Der Ausdruck "wirksarne Menge" bedeutet, dass die Wassermenge so hoch ist, dass eine spürbare Verbesserung der Selektivität im Vergleich zu einer Reaktion unter wasserfreien Bedingungen erzielt wird. Vorzugsweise wird daher Wasser der Reaktionsmischung hinzugefügt. Bei der Verwendung von Edukten mit einem besonders hohen Wasseranteil kann jedoch auf einen Wasserzusatz verzichtet werden.

Im Allgemeinen liegt die Menge an Wasser, bezogen auf das Gewicht des eingesetzten Butandiols, im Bereich von 0,005 bis 8 Gew.-%, vorzugsweise im Bereich von 0,01 bis 4 Gew.-% und besonders bevorzugt im Bereich von 0,1 bis 1 Gew.-%.

Gemäß einem besonderen Aspekt des vorliegenden Verfahrens kann das Verhältnis des Wassergewichts zum Gewicht des Katalysators im Bereich von 10:1 bis 1:10, bevorzugt 5:1 bis 1:5 und besonders bevorzugt im Bereich von 2:1 bis 1:2 liegen.

Wie bereits erwähnt, umfasst der erfindungsgemäße Katalysator mindestens eine Lithium- und mindestens eine Calciumverbindung. Bezogen auf das Gewicht der einzelnen Komponenten ergeben sich bevorzugt folgende Wasseranteile, ohne dass hierdurch eine Beschränkung erfolgen soll. Beispielsweise kann das Gewichtsverhältnis des Wassers zur Lithiumverbindung im Bereich von 20:1 bis 1:1, bevorzugt 10:1 bis 2:1 und das Gewichtsverhältnis des Wassers zur Calciumverbindung im Bereich von 10:1 bis 1:2, besonders bevorzugt 5:1 bis 1:1,75 liegen.

Erfindungsgemäß erfolgt zumindest ein Teil der Umsetzung in Gegenwart von Wasser. Dementsprechend ist es nicht notwendig, dass die oben genannten Mengenangaben über den gesamten Verlauf der Umesterung eingehalten werden. Vielmehr beziehen sich diese Angaben bei Chargenverfahren auf den Wasseranteil zu Beginn der Reaktion. Bei kontinuierlichen Umesterungsreaktionen können sich diese Angaben auf die zugegebene Eduktmischung beziehen. Hierbei ist zu berücksichtigen, dass ein Teil des Wassers üblich beispielsweise durch Bildung von Azeotropen zusammen mit den Alkoholen und/oder den eingesetzten Estern der Methacrylsäure aus der Reaktionsmischung im Verlaufe der Umsetzung abgetrennt werden kann. Daher kann der Wasseranteil über den Reaktionsverlauf abnehmen.

Die Umsetzung kann bei Über- oder Unterdruck erfolgen. Gemäß einer besonders zweckmäßigen Abwandlung der vorliegenden Erfindung kann die Umesterung bei einem Druck im Bereich von 200 bis 2000 mbar, besonders bevorzugt im Bereich von 500 bis 1300 mbar durchgeführt werden.

Die Reaktionstemperatur kann, insbesondere in Abhängigkeit des Druckes, ebenfalls in einem weiten Bereich liegen. Gemäß einer bevorzugten Ausführungsform der vorliegenden Erfindung erfolgt die Umsetzung vorzugsweise bei einer Temperatur im Bereich von 60°C bis 150°C, besonders bevorzugt im Bereich von 80°C bis 140°C und ganz besonders bevorzugt 90 bis 130 °C.

Überraschend können besondere Vorteile erzielt werden, falls die Temperatur, bei der die Umsetzung erfolgt, im Verlauf der Umsetzung erhöht wird. Gemäß dieser bevorzugten Abwandlung des erfindungsgemäßen Verfahrens kann die Temperatur zu Beginn der Umsetzung, insbesondere bis zu einem Umsatz von 80%, bevorzugt bis zu einem Umsatz von 70%, bezogen auf das Gewicht des eingesetzten Butandiols, vorzugsweise im Bereich von 90°C bis 110°C und gegen Ende der Umsetzung, insbesondere nach einem Umsatz von 80%, bevorzugt nach einem Umsatz von 90%, bezogen auf das Gewicht des eingesetzten Butandiols, im Bereich von 115 °C bis 130 °C liegen.

Die Umesterung kann sowohl kontinuierlich als auch chargenweise durchgeführt werden. Das erfindungsgemäße Verfahren kann in Substanz, d.h. ohne Verwendung eines weiteren Lösungsmittels durchgeführt werden. Falls gewünscht kann auch ein inertes Lösungsmittel eingesetzt werden. Hierzu gehören unter anderem Benzin, Benzol, Toluol, n-Hexan, Cyclohexan und Methylisobutylketon (MIBK), Methylethylketon (MEK).

Bei einer besonders zweckmäßigen Varianten der erfindungsgemäßen Umesterung werden sämtliche Komponenten, wie beispielsweise das Butandiol, der Methacrylsäureester sowie der Katalysator, gemischt, wonach diese Reaktionsmischung bis zum Sieden erwärmt wird. Anschließend kann der frei werdende Alkohol destillativ, beispielsweise Methanol oder Ethanol, gegebenenfalls azeotrop mit Methylmethacrylat oder Ethylmethacrylat, aus der Reaktionsmischung entfernt werden.

Die Reaktionszeiten sind unter anderem von den gewählten Parametern, wie zum Beispiel Druck und Temperatur, abhängig. Sie liegen aber im Allgemeinen im Bereich von 1 bis 24 Stunden, bevorzugt von 2 bis 20 Stunden und ganz besonders bevorzugt 6 bis 9 Stunden. Bei kontinuierlichen Verfahren liegen die Verweilzeiten im Allgemeinen im Bereich von 0,5 bis 24 Stunden, bevorzugt von 1 bis 12 Stunden und ganz besonders bevorzugt 2 bis 3 Stunden. Weitere Hinweise in Bezug auf die Reaktionszeiten kann der Fachmann den beigefügten Beispielen entnehmen.

Vorzugsweise kann die Reaktion unter Rühren stattfinden, wobei die Rührgeschwindigkeit besonders bevorzugt im Bereich von 50 bis 2000 Upm, ganz besonders bevorzugt im Bereich von 100 bis 500 Upm liegen kann.

Der pH-Wert kann in einem weiten Bereich liegen. Zweckmäßig kann die Umsetzung bei einem pH-Wert im Bereich von 8 bis 14, vorzugsweise 9 bis 13 durchgeführt werden.

Um eine unerwünschte Polymerisation der Methacrylate zu verhindern, können bei der Umsetzung Polymerisationsinhibitoren eingesetzt werden. Diese Verbindungen, wie beispielsweise Hydrochinone, Hydrochinonether, wie Hydrochinonmonomethylether oder Di-tert-butylbrenzcatechin, Phenothiazin, 4-Hydroxy-2,2,6,6-tetramethylpiperidin-1-oxyl, N,N'-Diphenyl-p-phenylendiamin, Methylenblau oder sterisch gehinderte Phenole, sind in der Fachwelt weithin bekannt. Diese Verbindungen können einzeln oder in Form von Mischungen eingesetzt werden und sind im Allgemeinen kommerziell erhältlich. Die Wirkung der Stabilisatoren besteht meist darin, dass sie als Radikalfänger für die bei der Polymerisation auftretenden freien Radikale wirken. Für weitere Details wird auf die gängige Fachliteratur, insbesondere auf das Römpp-Lexikon Chemie; Herausgeber: J. Falbe, M. Regitz; Stuttgart, New York; 10. Auflage (1996); Stichwort "Antioxidantien" und die an dieser Stelle zitierten Literaturstellen verwiesen. Vorzugsweise werden insbesondere Phenole als Polymerisationsinhibitor eingesetzt. Besonders überraschende Vorteile können bei Verwendung von Hydrochinonmonomethylether erzielt werden. Bezogen auf das Gewicht der gesamten Reaktionsmischung kann der Anteil der Inhibitoren einzeln oder als Mischung im Allgemeinen 0,01 - 0,5 % (wt/wt) betragen.

Diese Polymerisationsinhibitoren können vor oder zu Beginn der Reaktion in die Reaktionsmischung gegeben werden. Darüber hinaus können auch Teile der beigefügten Polymerisationsinhibitoren während der Umesterung hinzu gegeben werden. Von besonderem Interesse sind hierbei insbesondere Verfahren bei denen ein Teil des Polymerisationsinhibitors über den Kolonnenrücklauf zugefügt wird. Durch diese Maßnahme kann insbesondere eine unerwünschte Polymerisation innerhalb der Destillationskolonne vermieden werden.

Zur Inhibierung kann des Weiteren Sauerstoff verwendet werden: Hierbei kann dieser zum Beispiel in Form von Luft eingesetzt werden, wobei die Mengen vorteilhaft so dosiert werden, dass der Gehalt in der Gasphase über dem Reaktionsgemisch unterhalb der Explosionsgrenze bleibt. Besonders bevorzugt sind hierbei Sauerstoffmengen im Bereich von 0,05 bis 0,5 I pro Stunde und Mol Butandiol. Bei Chargenverfahren kann sich diese Menge auf die ursprünglich eingesetzte Menge an Butandiol beziehen. Bei kontinuierlichen Verfahren kann sich diese Menge auf die zugeführte Menge an Butandiol beziehen.

Gemäß einer besonderen Ausgestaltung der vorliegenden Erfindung kann zur Inhibierung eine Kombination von Sauerstoff mit mindestens einem Phenol, vorzugsweise Hydrochinonmonomethylether, eingesetzt werden. Ebenso können Inertgas-Sauerstoff-Gemische, z.B. Stickstoff-Sauerstoff- oder Argon-Sauerstoff - Gemische eingesetzt werden.

Entsprechend einer zweckmäßigen Ausführungsform der vorliegenden Erfindung kann der aus dem eingesetzten Methacrylat freigesetzte Alkohol, beispielsweise Methanol und/oder Ethanol, durch Destillation abgetrennt werden. Hierbei kann vorteilhaft beispielsweise eine Mischung abgetrennt werden, die Methylmethacrylat und Methanol enthält. Überraschend kann mit Vorteil ein Teil der abgetrennten Mischung in den folgenden Ansatz zurückgeführt werden. Gemäß dieser Abwandlung kann der zurückführbare Anteil der abgetrennten Mischung zu Ende der Reaktion, insbesondere nach einem Umsatz von 80%, bevorzugt nach einem Umsatz von 90% des eingesetzten Butandiols, erhalten werden. Beispielsweise kann der Anteil der zurückgeführten Mischung zu Beginn des folgenden Ansatzes im Bereich von 40 bis 60%, bezogen auf die Gesamteinwaage an umzuesterndem Methacrylsäureester liegen.

Von besonderem Interesse sind unter anderem Chargenverfahren, bei denen während der Umesterung Methylmethacrylat zugegeben wird. Diese Ausführungsform ist beispielsweise von Vorteil, falls Methylmethacrylat zusammen mit Methanol aus der Reaktionsmischung entfernt wird. Vorzugsweise kann das Gewichtsverhältnis der Menge an während der Umesterung zugegebenem Methylmethacrylat zur Menge an abgetrenntem Methanol-Methylmethacrylat-Gemisch im Bereich von 2:1 bis 1:3 liegen.

Bei Chargenverfahren kann gegen Ende der Reaktion überschüssiges Edukt, insbesondere der nicht umgesetzte Ester der Methacrylsäure durch Destillation abgetrennt werden. Auch dieser kann im nächsten Batch ohne weitere Reinigung wieder eingesetzt werden.

Das zu Beginn der Umsetzung erhaltene methanol- oder ethanolreiche Destillat kann ebenfalls recycliert werden, beispielsweise durch Einarbeitung in eine im Verbund betriebene Anlage zur Herstellung des umzuesternden Methacrylatesters.

Eine geeignete Anlage zur Durchführung der vorliegenden Umesterung kann beispielsweise einen Rührkesselreaktor mit Rührwerk, Dampfheizung, Destillationskolonne und Kondensator umfassen. Derartige Anlagen sind an sich bekannt und beispielsweise in Ullmanns Encyclopedia of Industrial Chemistry (6. Auflage), Verlag Wiley-VCH ,Weinheim 2003, Band 10, Seite 647, beschrieben. Die Größe der Anlage ist von der herzustellenden Menge an Butandioldimethacrylat abhängig, wobei das vorliegende Verfahren sowohl im Labormaßstab als auch in großtechnischem Maßstab durchgeführt werden kann. Gemäß einem besonderen Aspekt kann der Rührkesselreaktor dementsprechend ein Kesselvolumen im Bereich von 1m³ bis 30 m³, bevorzugt 3 m³ bis 20 m³ aufweisen. Das Rührwerk des Reaktorkessels kann insbesondere in Form eines Ankerrührers, Impellers, Schaufel- oder Inter-MIG-rührers ausgestaltet sein.

Die Aufgabe der Destillationskolonne ist es, sicherzustellen, daß ein methanol-bzw. ethanolreiches Azeotrop abgeführt wird, um die Verluste an zwangsläufig mit ausgetragenem Eduktester zu minimieren. Die Destillationskolonne kann ein, zwei oder mehr Trennstufen besitzen. Als Anzahl der Trennstufen wird die Anzahl der Böden bei einer Bodenkolonne oder die Anzahl der theoretischen Trennstufen im Fall einer Packungskolonne oder eine Kolonne mit Füllkörpern bezeichnet. Beispiele für eine mehrstufige Destillationskolonne mit Böden beinhalten solche wie Glockenböden, Siebböden, Tunnelböden, Ventilböden, Schlitzböden, Sieb-Schlitzböden, Sieb-Glockenböden, Düsenböden, Zentrifugalböden, für eine mehrstufige Destillationskolonne mit Füllkörpern solche wie Raschig-Ringe, Lessing-Ringe, Pall-Ringe, Berl-Sättel, Intalox Sättel und für eine mehrstufige Destillationskolonne mit Packungen wie solche vom Typ Mellapak (Sulzer), Rombopak (Kühni), Montz-Pak (Montz). Durch die umsatzabhängige Anpassung des Rücklaufverhältnis gelingt es beispielsweise, bei Verwendung von Methylmethacrylat über weite Bereiche des Umsatzes einen Methanolanteil im Destillat einzustellen, der oberhalb von 60% liegt.

Zu den geeigneten Kondensatoren, die in der Anlage zur Durchführung der vorliegenden Umesterung enthalten sein können, gehören unter anderem Platten- und Rohrbündelwärmetauscher.

Nach Beendigung der Umsetzung genügt das erhaltene Butandioldimethacrylat bereits vielfach den zuvor dargelegten hohen Anforderungen, so dass eine weitere Aufreinigung vielfach nicht notwendig ist. Zur weiteren Qualitätssteigerung und insbesondere der Katalysatorabtrennung kann die erhaltene Mischung durch bekannte Verfahren aufgereinigt werden.

Gemäß einer Ausgestaltung des erfindungsgemäßen Verfahrens kann die erhaltene Produktmischung durch Filtrationsverfahren aufgereinigt werden. Diese Verfahren sind aus dem Stand der Technik bekannt (W. Gösele, Chr. Alt in Ullmann's Encyclopedia of Industrial Chemistry, (6. Auflage),Verlag Wiley-VCH, Weinheim 2003 , Band 13, Seiten 731 und 746), wobei übliche Filtrationshilfsmittel, wie beispielsweise Bleicherde und Aluminiumsilicate (Perlit) eingesetzt werden können. Beispielsweise können unter anderem kontinuierlich betreibbare Filter für eine Anschwemmfiltration verwendet werden.

Eine weitere Verbesserung der Qualität des Produkts kann beispielsweise durch eine Niedersiederabtrennung aus dem erhaltenen Filtrat erzielt werden, wobei zum Beispiel ein kontinuierlicher Verdampfer mit rotierendem Wischersystem und aufgesetzter Kolonne eingesetzt werden kann. Solche Vorrichtungen sind bekannt (Ullmanns Encyclopedia of Industrial Chemistry (6. Auflage), Verlag Wiley-VCH, Weinheim 2003, Band 36, Seite 505). Diese Destillation kann beispielsweise bei Druck im Bereich von 5 bis 60 mbar und einer Verdampfertemperatur von 120°C bis 150°C durchgeführt werden.

Nachfolgend soll die vorliegende Erfindung anhand von Beispielen und Vergleichsbeispielen erläutert werden, ohne dass hierdurch eine Beschränkung erfolgen soll.

### Beispiel 1

In einem 6m³ - Rührkesselreaktor mit Rührwerk, Dampfheizung, Destillationskolonne und Kondensator werden 788 kg 1,4-Butandiol, 2664 kg Methacrylsäuremethylester (MMA), 0,123 kg Hydrochinonmonomethylether als Inhibitor und als Katalysator ein Gemisch aus 5 kg Calciumoxid, 1 kg Lithiumhydroxid und 4 kg Wasser vereint und unter Einleiten von Luft gerührt. Zur Kolonnenstabilisierung werden im Laufe der Reaktion insgesamt 151 kg MMA, die 0,24 kg Hydrochinonmonomethylether und 0,016 kg 4-Hydroxy-2,2,6,6-tetramethylpiperidin-1-oxyl gelöst enthalten in den Kolonnenrücklauf dosiert. Man erhitzt auf 96°C Sumpftemperatur, wobei die Kolonne zunächst unter vollständigem Rücklauf betrieben wird. Sobald die Temperatur am Kolonnenkopf unter 70°C fällt, zieht man unter einem Rücklaufverhältnis von 2,5 :1 das Methanol-MMA-Gemisch ab. Dabei ergänzt man den MMA-Vorrat im Reaktor durch Zudosieren von gleichen Teilen MMA pro Teil abgezogenem Methanol-MMA-Gemisch. Innerhalb von 5 h werden so insgesamt 1267 kg MMA eingebracht. Innerhalb von 8 h wird das Rücklaufverhältnis bis auf 4,5:1 der abnehmenden Methanolentwicklung angepaßt. Bei einer Sumpftemperatur von 130 °C ist die Reaktion beendet und man zieht überschüssiges MMA im Vakuum ab, wobei man den Druck allmählich bis auf 100 mbar reduziert. Wenn kein MMA mehr abdestilliert, wird das Vakuum aufgehoben. Der Kesselinhalt, bestehend aus dem katalysatorhaltigen 1,4-Butandioldimethacrylat wird mit 9 kg Bleicherde und 6 kg Aluminiumsilicat (Perlit) als Filterhilfsmittel versetzt und per Anschwemmfiltration vom Katalysator befreit. Das Filtrat wird in einen kontinuierlichen Verdampfer (Fläche 2 m²) mit rotierendem Wischersystem bei 15 Torr Druck und 142°C Verdampfertemperatur eingespeist. Aus dem Sumpfaustrag erhält man insgesamt 1800 kg 1,4-Butandioldimethacrylat: Zusammensetzung (gaschromatographisch ermittelt):

| | |
|---|---|
| 93,5 % | 1,4-Butandioldimethacrylat |
| 0,05 % | MMA |
| 0,1 % | 1,4-Butandiolmonomethacrylat |
| 2,08 % | 4-(Methacryloyloxy)butyl(3'-(methoxy)isobutyrat) ( 4-(3-Methoxy-2-methylpropanoyloxy)butyl 2-methylprop-2-enoat gemäß IUPAC) |
| 2,6% | 4-(Methacryloyloxy)butyl(3'-(4"-(methacryloyloxy)butyl)isobutyrat) (4-(2-{[4-(2-methylprop-2-enoyloxy)butyl]oxycarbonyl}propoxy)butyl 2-methylprop-2-enoat gemäß IUPAC) |

### Beispiel 2

In einem 6m³ - Rührkesselreaktor mit Rührwerk, Dampfheizung, Destillationskolonne und Kondensator werden 792 kg 1,3-Butandiol, 2650 kg Methacrylsäuremethylester (MMA), 0,125kg Hydrochinonmonomethylether als Inhibitor und als Katalysator ein Gemisch aus 5 kg Calciumoxid, 1 kg Lithiumhydroxid und 3 kg Wasser vereint und unter Einleiten von Luft gerührt. Zur Kolonnenstabilisierung werden im Laufe der Reaktion insgesamt 151 kg MMA, die 0,24 kg Hydrochinonmonomethylether und 0,016 kg 4-Hydroxy-2,2,6,6-tetramethylpiperidin-1-oxyl gelöst enthalten in den Kolonnenrücklauf dosiert. Man erhitzt auf 100°C Sumpftemperatur, wobei die Kolonne zunächst unter vollständigem Rücklauf betrieben wird. Sobald die Temperatur am Kolonnenkopf unter 70°C fällt, zieht man unter einem Rücklaufverhältnis von 4 :1 das Methanol-MMA-Gemisch ab. Dabei ergänzt man den MMA-Vorrat im Reaktor durch Zudosieren von gleichen Teilen frischem MMA pro Teil abgezogenem Methanol-MMA-Gemisch. Innerhalb von 5 h werden so insgesamt 1226 kg MMA eingebracht. Innerhalb von 8 h wird das Rücklaufverhältnis bis auf 1,5:1 der abnehmenden Methanolentwicklung angepaßt. Bei einer Sumpftemperatur von 130 °C ist die Reaktion beendet und man zieht überschüssiges MMA im Vakuum ab, wobei man den Druck allmählich bis auf 100 mbar reduziert. Wenn kein MMA mehr abdestilliert, wird das Vakuum aufgehoben. Der Kesselinhalt, bestehend aus dem katalysatorhaltigen 1,3-Butandioldimethacrylat wird mit 9 kg Bleicherde und 6 kg Aluminiumsilicat (Perlit) als Filterhilfsmittel versetzt und per Anschwemmfiltration vom Katalysator befreit. Das Filtrat wird in einen kontinuierlichen Verdampfer (Fläche 3,5 m²) mit rotierendem Wischersystem bei 18 Torr Druck und 134°C Verdampfertemperatur eingespeist. Aus dem Sumpfaustrag erhält man insgesamt 1810 kg 1,3-Butandioldimethacrylat: Zusammensetzung (gaschromatographisch ermittelt):

| | |
|---|---|
| 93,6 % | 1,3-Butandioldimethacrylat |
| 0,1 % | Methylmethacrylat (MMA) |
| 0,66 % | 1,3-Butandiolmonomethacrylat |
| 1,8 % | 3-(Methacryloyloxy)butyl(3-(methoxy)isobutyrat) (3-(3-methoxy-2-methylpropanoyloxy)-1-methylpropyl 2-methylprop-2-enoat gemäß IUPAC) |
| 2,9 % | 3-(Methacryloyloxy)butyl(3'-(3"-(methacryloyloxy)butyl)isobutyrat) 1-methyl-3-(2-{[3-(2-methylprop-2-enoyloxy)butyl]oxycarbonyl} propoxy)propyl 2-methylprop-2-enoat (IUPAC) |

### Vergleichsbeispiel 1

Das obige Beispiel 1 wurde im Wesentlichen wiederholt, wobei jedoch kein Wasser dem Katalysatorgemisch zugesetzt wurde. Man erhält 1790 kg 1,4-Butandioldimethacrylat mit folgender Zusammensetzung (gaschromatographisch ermittelt):

| | |
|---|---|
| 87,6 % | 1,4-Butandioldimethacrylat |
| 0,2 % | MMA |
| 0,05 % | 1,4-Butandiolmonomethacrylat |
| 6,1 % | 4-(Methacryloyloxy)butyl(3'-(methoxy)isobutyrat) |
| 5,1 % | 4-(Methacryloyloxy)butyl(3'-(4"-(methacryloyloxy)butyl)isobutyrat) |

### Vergleichsbeispiel 2

Das obige Beispiel 2 wurde im Wesentlichen wiederholt, wobei jedoch kein Wasser dem Katalysatorgemisch zugesetzt wurde. Man erhält 1805 kg 1,3-Butandioldimethacrylat mit folgender Zusammensetzung (gaschromatographisch ermittelt):

| | |
|---|---|
| 90,4 % | 1,3-Butandioldimethacrylat |
| 0,25 % | MMA |
| 0,7 % | 1,3-Butandiolmonomethacrylat |
| 3,5 % | 3-(Methacryloyloxy)butyl(3'-(methoxy)isobutyrat) |
| 4,6 % | 3-(Methacryloyloxy)butyl(3'-(3"-(methacryloyloxy)butyl)isobutyrat) |

### Vergleichsbeispiel 3: Herstellung ohne Wasserzusatz, aber mit Calciumhydroxid an Stelle von Calciumoxid

In einem 6m³ - Rührkesselreaktor mit Rührwerk, Dampfheizung, Destillationskolonne und Kondensator werden 788 kg 1,4-Butandiol, 2664 kg Methacrylsäuremethylester (MMA) bestehend aus 1155 kg frischem MMA und 1509 kg recycliertem MMA aus der Vakuumdestillationsphase von Vergleichsbeispiel 1, 0,123 kg Hydrochinonmonomethylether als Inhibitor und als Katalysator ein Gemisch aus 3,3 kg Calciumhydroxid und 0,5 kg Lithiumhydroxid vereint und unter Einleiten von Luft gerührt. Zur Kolonnenstabilisierung werden im Laufe der Reaktion insgesamt 151 kg MMA, die 0,24 kg Hydrochinonmonomethylether und 0,016 kg 4-Hydroxy-2,2,6,6-tetramethylpiperidin-1-oxyl gelöst enthalten in den Kolonnenrücklauf dosiert. Man erhitzt auf 91 °C Sumpftemperatur, wobei die Kolonne zunächst unter vollständigem Rücklauf betrieben wird. Sobald die Temperatur am Kolonnenkopf unter 70°C fällt, zieht man unter einem Rücklaufverhältnis von 3 :1 das Methanol-MMA-Gemisch ab. Dabei ergänzt man den MMA-Vorrat im Reaktor durch Zudosieren von gleichen Teilen MMA pro Teil abgezogenem Methanol-MMA-Gemisch. Innerhalb von 5 h werden so insgesamt 1267 kg MMA eingebracht. Innerhalb von 8 h wird das Rücklaufverhältnis bis auf 6,5:1 der abnehmenden Methanolentwicklung angepaßt. Bei einer Sumpftemperatur von 130 °C ist die Reaktion beendet, die Kolonnenkopftemperatur beträgt nun 101°C und man zieht anschließend überschüssiges MMA im Vakuum ab, wobei man den Druck allmählich bis auf 100 mbar reduziert. Wenn kein MMA mehr abdestilliert, wird das Vakuum aufgehoben. Der Kesselinhalt, bestehend aus dem katalysatorhaltigen 1,4-Butandioldimethacrylat wird mit 9 kg Bleicherde und 6 kg Aluminiumsilicat (Perlit) als Filterhilfsmittel versetzt und per Anschwemmfiltration vom Katalysator befreit. Das Filtrat wird in einen kontinuierlichen Verdampfer (Fläche 2 m²) mit rotierendem Wischersystem bei 15 Torr Druck und 142°C Verdampfertemperatur eingespeist.

Aus dem Sumpfaustrag erhält man insgesamt 1806 kg 1,4-Butandioldimethacrylat:
Zusammensetzung (gaschromatographisch ermittelt):
   82,2 % 1,4-Butandioldimethacrylat
   0,1 % MMA
   0,2 % 1,4-Butandiolmonomethacrylat
   8,1 % 4-(Methacryloyloxy)butyl(3'-(methoxy)isobutyrat)
   4-((3-Methoxy-2-methylpropanoyloxy)butyl 2-methylprop-2-enoat (IUPAC)
   6,1 % 4-(Methacryloyloxy)butyl(3'-(4"-(methacryloyloxy)butyl)isobutyrat)
   4-((2-{[4-(2-methylprop-2-enoyloxy)butyl]oxycarbonyl}propoxy)butyl 2-methylprop-2-enoat (IUPAC)

### Vergleichsbeispiel 4: Herstellung ohne Wasserzusatz, aber mit Calciumhydroxid an Stelle von Calciumoxid

In einem 6m³ - Rührkesselreaktor mit Rührwerk, Dampfheizung, Destillationskolonne und Kondensator werden 792 kg 1,3-Butandiol, 2655 kg Methacrylsäuremethylester (MMA) bestehend aus 1150 kg frischem MMA und 1505 kg recycliertem MMA aus der Vakuumdestillationsphase von Vergleichsbeispiel 2, 0,125kg Hydrochinonmonomethylether als Inhibitor und als Katalysator ein Gemisch aus 3,3 kg Calciumhydroxid und 0,5 kg Lithiumhydroxid vereint und unter Einleiten von Luft gerührt. Zur Kolonnenstabilisierung werden im Laufe der Reaktion insgesamt 151 kg MMA, die 0,24 kg Hydrochinonmonomethylether und 0,016 kg 4-Hydroxy-2,2,6,6-tetramethylpiperidin-1-oxyl gelöst enthalten in den Kolonnenrücklauf dosiert. Man erhitzt auf 100°C Sumpftemperatur, wobei die Kolonne zunächst unter vollständigem Rücklauf betrieben wird. Sobald die Temperatur am Kolonnenkopf unter 70°C fällt, zieht man unter einem Rücklaufverhältnis von 4 :1 das Methanol-MMA-Gemisch ab. Dabei ergänzt man den MMA-Vorrat im Reaktor durch Zudosieren von gleichen Teilen frischem MMA pro Teil abgezogenem Methanol-MMA-Gemisch. Innerhalb von 5 h werden so insgesamt 1226 kg MMA eingebracht. Innerhalb von 8 h wird das Rücklaufverhältnis bis auf 1,5:1 der abnehmenden Methanolentwicklung angepaßt. Bei einer Sumpftemperatur von 130 °C ist die Reaktion beendet, die Kolonnenkopftemperatur beträgt nun 100°C und man zieht überschüssiges MMA im Vakuum ab, wobei man den Druck allmählich bis auf 100 mbar reduziert. Wenn kein MMA mehr abdestilliert, wird das Vakuum aufgehoben. Der Kesselinhalt, bestehend aus dem katalysatorhaltigen 1,3-Butandioldimethacrylat wird mit 9 kg Bleicherde und 6 kg Aluminiumsilicat (Perlit) als Filterhilfsmittel versetzt und per Anschwemmfiltration vom Katalysator befreit.

Das Filtrat wird in einen kontinuierlichen Verdampfer (Fläche 3,5 m²) mit rotierendem Wischersystem bei 18 Torr Druck und 134°C Verdampfertemperatur eingespeist.

Aus dem Sumpfaustrag erhält man insgesamt 1815 kg 1,3-Butandioldimethacrylat:
Zusammensetzung (gaschromatographisch ermittelt):
   84,2 % 1,3-Butandioldimethacrylat
   0,15 % MMA
   0,12 % 1,3-Butandiolmonomethacrylat
   6,3 % 3-(Methacryloyloxy)butyl(3-(methoxy)isobutyrat)
   [3-((3-methoxy-2-methylpropanoyloxy)-1-methylpropyl 2-methylprop-2-enoat ] (IUPAC)
   6,4 % 3-(Methacryloyloxy)butyl(3'-(3"-(methacryloyloxy)butyl)isobutyrat)
   1-methyl-3-(2-{[3-(2-methylprop-2-enoyloxy)butyl]oxycarbonyl}propoxy)propyl 2-methylprop-2-enoat (IUPAC)

## Patentansprüche

1. Verfahren zur Herstellung von Butandioldimethacrylaten, umfassend die Umesterung von Butandiolen mit einem Ester der Methacrylsäure in Gegenwart von Katalysatoren, **dadurch gekennzeichnet, dass** als Katalysator eine Kombination eingesetzt wird, die mindestens eine Lithiumverbindung und mindestens eine Calciumverbindung umfasst, wobei mindestens eine der Verbindungen des Lithiums und/oder des Calciums ein Oxid, ein Hydroxid, ein Alkoxid mit 1 bis 4 Kohlenstoffatomen oder ein Carboxylat mit 1 bis 4 Kohlenstoffatomen ist und zumindest ein Teil der Umsetzung in Gegenwart einer wirksamen Menge an Wasser erfolgt und die Menge an Wasser, bezogen auf das Gewicht des eingesetzten Butandiols, im Bereich von 0,01 bis 4 Gew.-% liegt, wobei das Wasser der Reaktionsmischung hinzu gegeben wird.

2. Verfahren gemäß Anspruch 1, **dadurch gekennzeichnet, dass** 1,4-Butandiol oder 1,3-Butandiol eingesetzt wird.

3. Verfahren gemäß mindestens einem der vorhergehenden Ansprüche, **dadurch gekennzeichnet, dass** das Verhältnis des Wassergewichts zum Gewicht des Katalysators im Bereich von 5:1 bis 1:5 liegt und/oder dass das Verhältnis des Wassergewichts zum Gewicht der Lithiumverbindung im Bereich von 20:1 bis 1:1 liegt und/oder dass das Verhältnis des Wassergewichts zum Gewicht der Calciumverbindung im Bereich von 10:1 bis 1:2 liegt.

4. Verfahren gemäß mindestens einem der vorhergehenden Ansprüche, **dadurch gekennzeichnet, dass** als Lithiumverbindung Lithiumhydroxid, Lithiumoxid, Lithiummethanolat und/oder Lithiumethanolat und/oder dass als Calciumverbindung des Calciumoxid, Calciumhydroxid, Calciummethanolat und/oder Calciumethanolat eingesetzt wird.

5. Verfahren gemäß mindestens einem der vorhergehenden Ansprüche, **dadurch gekennzeichnet, dass** als Katalysator eine Mischung eingesetzt wird, die Lithiumhydroxid und Calciumoxid umfasst und / oder dass als Katalysator eine Mischung eingesetzt wird, die Lithiumhydroxid und Calciumhydroxid umfasst.

6. Verfahren gemäß mindestens einem der vorhergehenden Ansprüche, **dadurch gekennzeichnet, dass** das Verhältnis des Gewichts der Lithiumverbindung zum Gewicht der Calciumverbindung im Bereich von 20:1 bis 1:20 liegt.

7. Verfahren gemäß mindestens einem der vorhergehenden Ansprüche, **dadurch gekennzeichnet, dass** die Reaktionszeit im Bereich von 2 bis 20 Stunden liegt.

8. Verfahren gemäß mindestens einem der vorhergehenden Ansprüche, **dadurch gekennzeichnet, dass** Methylmethacrylat oder Ethylmethacrylat als Ester der Methacrylsäure eingesetzt wird.

9. Verfahren gemäß mindestens einem der vorhergehenden Ansprüche, **dadurch gekennzeichnet, dass** das Gewichtsverhältnis von Butandiol zum Ester der Methacrylsäure im Bereich von 1:2 bis 1:20 liegt.

10. Verfahren gemäß mindestens einem der vorhergehenden Ansprüche, **dadurch gekennzeichnet, dass** die Umsetzung bei einem Druck im Bereich von 500 bis 1.300 mbar erfolgt.

11. Verfahren gemäß Anspruch 9 oder 10, **dadurch gekennzeichnet, dass** die Temperatur zu Beginn der Umsetzung im Bereich von 90°C bis 110°C und gegen Ende der Umsetzung im Bereich von 115 °C bis 130 °C liegt.

12. Verfahren gemäß mindestens einem der vorhergehenden Ansprüche, **dadurch gekennzeichnet, dass** die Umsetzung in Gegenwart eines Polymerisationsinhibitors und / oder unter Einleitung von Luftsauerstoff erfolgt.

13. Verfahren gemäß Anspruch 12, **dadurch gekennzeichnet, dass** ein Phenol als Polymerisationsinhibitor eingesetzt wird.

14. Verfahren gemäß mindestens einem der vorhergehenden Ansprüche, **dadurch gekennzeichnet, dass** der aus dem eingesetzten Ester der Methacrylsäure freigesetzte Alkohol durch Destillation abgetrennt wird.

15. Verfahren gemäß Anspruch 14, **dadurch gekennzeichnet, dass** ein Teil der abgetrennten Mischung in den folgenden Ansatz zurückgeführt wird.

16. Verfahren gemäß Anspruch 15, **dadurch gekennzeichnet, dass** der zurückführbare Anteil der abgetrennten Mischung gegen Ende der Reaktion gewonnen wird.

17. Verfahren gemäß einem der vorstehenden Ansprüche, **dadurch gekennzeichnet, dass** das Gewichtsverhältnis der Menge an während der Umesterung zugegebenem Methylmethacrylat zur Menge an abgetrenntem Methanol-Methylmethacrylat-Gemisch im Bereich von 2:1 bis 1:3 liegt.

## Claims

1. Process for preparing butanediol dimethacrylates, which comprises the transesterification of butanediols with an ester of methacrylic acid in the presence of catalysts, **characterized in that** a combination comprising at least one lithium compound and at least one calcium compound is used as catalyst, where at least one of the compounds of lithium and/or of calcium is an oxide, a hydroxide, an alkoxide having from 1 to 4 carbon atoms or a carboxylate having from 1 to 4 carbon atoms and at least part of the reaction is carried out in the presence of an effective amount of water and the amount of water, based on the weight of the butanediol used, is in the range from 0.01 to 4% by weight, where the water is added to the reaction mixture.

2. Process according to Claim 1, **characterized in that** 1,4-butandiol or 1,3-butanediol is used.

3. Process according to at least one of the preceding claims, **characterized in that** the ratio of the weight of water to the weight of the catalyst is in the range from 5:1 to 1:5 and/or **in that** the ratio of the weight of water to the weight of the lithium compound is in the range from 20:1 to 1:1 and/or **in that** the ratio of the weight of water to the weight of the calcium compound is in the range from 10:1 to 1:2.

4. Process according to at least one of the preceding claims, **characterized in that** lithium hydroxide, lithium oxide, lithium methoxide and/or lithium ethoxide is used as lithium compound and/or **in that** calcium oxide, calcium hydroxide, calcium methoxide and/or calcium ethoxide is used as calcium compound.

5. Process according to at least one of the preceding claims, **characterized in that** a mixture comprising lithium hydroxide and calcium oxide is used as catalyst and/or **in that** a mixture comprising lithium hydroxide and calcium hydroxide is used as catalyst.

6. Process according to at least one of the preceding claims, **characterized in that** the ratio of the weight of the lithium compound to the weight of the calcium compound is in the range from 20:1 to 1:20.

7. Process according to at least one of the preceding claims, **characterized in that** the reaction time is in the range from 2 to 20 hours.

8. Process according to at least one of the preceding claims, **characterized in that** methyl methacrylate or ethyl methacrylate is used as ester of methacrylic acid.

9. Process according to at least one of the preceding claims, **characterized in that** the weight ratio of butanediol to the ester of methacrylic acid is in the range from 1:2 to 1:20.

10. Process according to at least one of the preceding claims, **characterized in that** the reaction is carried out at a pressure in the range from 500 to 1300 mbar.

11. Process according to Claim 9 or 10, **characterized in that** the temperature at the beginning of the reaction is in the range from 90°C to 110°C and that towards the end of the reaction is in the range from 115°C to 130°C.

12. Process according to at least one of the preceding claims, **characterized in that** the reaction is carried out in the presence of a polymerization inhibitor and/or with introduction of oxygen.

13. Process according to Claim 12, **characterized in that** a phenol is used as polymerization inhibitor.

14. Process according to at least one of the preceding claims, **characterized in that** the alcohol liberated from the ester of methacrylic acid used is separated off by distillation.

15. Process according to Claim 14, **characterized in that** part of the mixture which has been separated off has been recirculated to the following batch.

16. Process according to Claim 15, **characterized in that** the proportion which can be recirculated of the mixture which has been separated off can be obtained at the end of the reaction.

17. Process according to any of the preceding claims, **characterized in that** the weight ratio of the amount of methyl methacrylate added during the transesterification to the amount of methanol/methyl methacrylate mixture separated off is in the range from 2:1 to 1:3.

## Revendications

1. Procédé pour la production de diméthacrylates de butanediol, comprenant la transestérification de butanediols par un ester de l'acide méthacrylique en présence de catalyseurs, **caractérisé en ce qu'**on utilise comme catalyseur une association qui comprend au moins un composé de lithium et au moins un composé de calcium, au moins l'un des composés du lithium et/ou du calcium étant un oxyde, un hydroxyde, un alcoolate ayant de 1 à 4 atomes de carbone ou un carboxylate ayant de 1 à 4 atomes de carbone et au moins une partie de la réaction s'effectuant en présence d'une quantité efficace d'eau et la quantité d'eau, par rapport au poids du butanediol utilisé, se situant dans la plage de 0,01 à 4 % en poids, l'eau étant ajoutée au mélange réactionnel.

2. Procédé selon la revendication 1, **caractérisé en ce qu'**on utilise du 1,4-butanediol ou du 1,3-butanediol.

3. Procédé selon au moins l'une quelconque des revendications précédentes, **caractérisé en ce que** le rapport du poids d'eau au poids du catalyseur se situe dans la plage de 5:1 à 1:5 et/ou **en ce que** le rapport du poids d'eau au poids du composé de lithium se situe dans la plage de 20:1 à 1:1 et/ou **en ce que** le rapport du poids d'eau au poids du composé de calcium se situe dans la plage de 10:1 à 1:2.

4. Procédé selon au moins l'une quelconque des revendications précédentes, **caractérisé en ce qu'**on utilise comme composé de lithium l'hydroxyde de lithium, l'oxyde de lithium, le méthanolate de lithium et/ou l'éthanolate de lithium et/ou comme composé de calcium l'oxyde de calcium, l'hydroxyde de calcium, le méthanolate de calcium et/ou l'éthanolate de calcium.

5. Procédé selon au moins l'une quelconque des revendications précédentes, **caractérisé en ce qu'**on utilise comme catalyseur un mélange qui comprend de l'hydroxyde de lithium et de l'oxyde de calcium et/ou on utilise comme catalyseur un mélange qui comprend de l'hydroxyde de lithium et de l'hydroxyde de calcium.

6. Procédé selon au moins l'une quelconque des revendications précédentes, **caractérisé en ce que** le rapport du poids du composé de lithium au poids du composé de calcium se situe dans la plage de 20:1 à 1:20.

7. Procédé selon au moins l'une quelconque des revendications précédentes, **caractérisé en ce que** le temps de réaction se situe dans la plage de 2 à 20 heures.

8. Procédé selon au moins l'une quelconque des revendications précédentes, **caractérisé en ce que** comme ester de l'acide méthacrylique on utilise le méthacrylate de méthyle ou le méthacrylate d'éthyle.

9. Procédé selon au moins l'une quelconque des revendications précédentes, **caractérisé en ce que** le rapport pondéral du butanediol à l'ester de l'acide méthacrylique se situe dans la plage de 1:2 à 1:20.

10. Procédé selon au moins l'une quelconque des revendications précédentes, **caractérisé en ce que** la réaction d'effectue sous une pression dans la plage de 500 à 1 300 mbars.

11. Procédé selon la revendication 9 ou 10, **caractérisé en ce que** la température se situe au début de la réaction dans la plage de 90 °C à 110 °C et vers la fin de la réaction dans la plage de 115 °C à 130 °C.

12. Procédé selon au moins l'une quelconque des revendications précédentes, **caractérisé en ce que** la réaction s'effectue en présence d'un inhibiteur de polymérisation et/ou avec introduction d'oxygène de l'air.

13. Procédé selon la revendication 12, **caractérisé en ce qu'**on utilise un phénol comme inhibiteur de polymérisation.

14. Procédé selon au moins l'une quelconque des revendications précédentes, **caractérisé en ce qu'**on sépare par distillation l'alcool libéré à partir de l'ester utilisé de l'acide méthacrylique.

15. Procédé selon la revendication 14, **caractérisé en ce qu'**une partie du mélange fractionné est recyclée dans la charge suivante.

16. Procédé selon la revendication 15, **caractérisé en ce que** la fraction recyclable du mélange fractionné est obtenue vers la fin de la réaction.

17. Procédé selon l'une quelconque des revendications précédentes, **caractérisé en ce que** le rapport pondéral de la quantité de méthacrylate de méthyle ajouté pendant la transestérification à la quantité du mélange méthanol-méthacrylate de méthyle fractionné se situe dans la plage de 2:1 à 1:3.
